# EUROPEAN PATENT APPLICATION

(11) **EP 4 837 564 A1**
(43) Date of publication of application: **11.11.2026**
(21) Application number: 24899920.3
(22) Date of filing: 05.12.2024
(51) Int. Cl.: A61K 9/06, A61P 27/02

(54) **NUCLEIC ACID HYDROGEL FORMULATION, AND USE THEREOF IN RETINA REATTACHMENT**

(30) Priority: 06.12.2023 CN 202311662264
(71) Applicant: Shanghai Clearvision Medical Technology Co., Ltd, Shanghai 200240 (CN)
(72) Inventor: HU, Chunchen, Suzhou, Jiangsu 215101 (CN); HE, Jian, Suzhou, Jiangsu 215101 (CN)
(74) Representative: Fuchs Patentanwälte Partnerschaft mbB
(86) International application number: PCT/CN2024/137153
(87) International publication number: WO 2025/119286

(57) **Abstract**

A pH-stable nucleic acid hydrogel formulation, a preparation method therefor, and a use of the nucleic acid hydrogel in retina reattachment with indentation.

## Description

### TECHNICAL FIELD

The present invention relates to a pH-stable nucleic acid hydrogel formulation, a preparation method therefor, and use of the nucleic acid hydrogel for retinal reattachment with indentation.

### BACKGROUND ART

Vitreous body is a clear gel-like substance filled in the posterior part of the eyeball and is very important for maintaining the shape of the eyeball, supporting the retina, and conducting light. The main physiological functions of the vitreous body include supporting the retina, ocular refraction, cellular barrier, and nutrition, etc. However, some ocular diseases, traumas, or age factors may cause problems of the vitreous body that require treatment or replacement. Clinically, pars plana vitrectomy (PPV) or vitrectomy is one of the effective means for treating severe ophthalmic diseases such as retinal detachment (RD), macular holes (MH), a diabetic retinopathy (DR)-related complication, and ocular trauma (OT) of the posterior segment.

In the PPV, it is necessary to excise part of the vitreous body and fill in the eyeball by injecting a special gas (such as sulfur hexafluoride and perfluoropropane) or silicone oil during the operation, thereby lifting and indenting the detached retina so as to make it reattach and gradually return to normal.

Although the gas has a good filling effect, it may cause significant visual deviation due to its relatively low refractive index and has a relatively short residence time in the body. Silicone oil has the advantages of good transparency, a refractive index close to the natural vitreous body, and high viscosity. However, the silicone oil is prone to emulsification in the intraocular environment, which may cause a series of complications after surgery, and its clinical use is limited. In the past decades, synthesized hydrogels have gradually attracted attention due to their high moisture content, good stability, and controllable mechanical properties. However, traditional chemically cross-linked hydrogels may destroy the network structure of the hydrogels during injection, resulting in their decreased mechanical properties and their uneven distribution in the vitreous chamber. Moreover, there is also a need for a safe method to decompose the traditional synthetic hydrogels and remove the hydrogels after the patient has recovered. Supramolecular hydrogels have unique shear thinning and self-healing properties compared to the traditional chemically cross-linked hydrogels, which can be conveniently injected directly and removed secondarily in a minimally invasive ophthalmic surgery. However, the supramolecular hydrogels, as vitreous replacement materials, have relatively poor mechanical strength and stability under dilution conditions.

Although a variety of hydrogels have been proved to have a potential to serve as vitreous replacement materials, how to construct more balanced vitreous replacement materials remains a challenge. Such materials need to have the characteristics such as reliable safety, long-term stability, good mechanical properties, and injectability at the same time.

Reference cited is as follows:
Yu Jin, Yujie Li, Sijia Song, Yuqiao Ding, Yuanchen Dong, Yao Lu, Dongsheng Liu, and Chun Zhang, DNA Supramolecular Hydrogel as a Biocompatible Artificial Vitreous Substitute. Adv. Mater. Interfaces 2021, 2101321.

### SUMMARY OF THE INVENTION

Based on the excellent biocompatibility of DNA hydrogels in eyes, the inventors of the present invention have found that a nucleic acid hydrogel formed by a nucleic acid hydrogel formulation composed of a certain solid content of nucleic acid hydrogel, a buffer, and optionally a pH adjusting agent has physicochemical properties similar to those of natural vitreous body. After the PPV surgery in a patient with a retinal detachment, injecting the nucleic acid hydrogel formulation can have sufficient indentation and supporting effects on the detached retina, thereby maintaining the shape of the eyeball. The inventors of the present invention have found that during preparation of the nucleic acid hydrogel formulation, adjusting purities of the single-stranded nucleic acids, pH of the system, gelation temperature, and different solid contents is crucial to the preparation process of the hydrogel and the mechanical properties at the time of filling, for example, a nucleic acid hydrogel having a stable pH value for a long term can be obtained by selecting a suitable buffer and/or adjusting the pH value, and a nucleic acid hydrogel formulation suitable for injection and retinal reattachment can be obtained by selecting a suitable solid content of the nucleic acids, and so on.

The present invention thereby provides a nucleic acid hydrogel formulation which is suitable for use in filling the vitreous chamber after the PPV surgery in patients with retinal detachment and assists in the treatment of retinal reattachment in patients with various retinal detachments.

In a first aspect, the present invention relates to a nucleic acid hydrogel formulation suitable for use in retinal reattachment, the nucleic acid hydrogel formulation comprising
a scaffold unit comprising a scaffold core and at least three single-stranded nucleic acids bound to the scaffold core, each single-stranded nucleic acid having at least one scaffold cohesive end;
a cross-linking unit comprising a cross-linking core and at least two single-stranded nucleic acids bound to the cross-linking core, each single-stranded nucleic acid having at least one cross-linking cohesive end; and
the scaffold units and the cross-linking units being cross-linked by the way of base complementary pairing of the scaffold cohesive ends and the cross-linking cohesive ends to form a nucleic acid hydrogel having a three-dimensional spatial network structure;
a buffer; and
optionally a pH adjusting agent.

In a second aspect, the present invention relates to a method for preparing a nucleic acid hydrogel formulation, the method comprising:
a) formulating a scaffold unit assembly solution and a cross-linking unit assembly solution; and
b) assembling the formulated scaffold unit assembly solution and the cross-linking unit assembly solution into a gel at a temperature higher than room temperature.

In an embodiment of the first aspect or the second aspect, the scaffold unit comprises a scaffold core and at least three single-stranded nucleic acids bound to the scaffold core, each single-stranded nucleic acid having at least one scaffold cohesive end. Preferably, the scaffold core is a nucleic acid, specifically may be a D-nucleic acid or an L-nucleic acid, more specifically may be a D-DNA or an L-DNA. In an embodiment of the first aspect or the second aspect, the nucleic acid serving as the scaffold core has a complementary pairing region which may be from about 4 bp to about 150 bp, preferably from about 5 bp to about 50 bp, more preferably from about 6 bp to about 30 bp, more preferably from about 8 bp to about 20 bp in length.

In an embodiment of the first aspect or the second aspect, the cross-linking unit comprises a cross-linking core and at least two single-stranded L-deoxynucleic acids bound to the cross-linking core, each single-stranded L-deoxynucleic acid having at least one cross-linking cohesive end. Preferably, the cross-linking core may be a deoxynucleic acid, specifically may be a D-nucleic acid or an L-nucleic acid, more specifically may be a D-DNA or an L-DNA. In an embodiment of the first aspect or the second aspect, the deoxynucleic acid serving as the cross-linking core has a complementary pairing region which may be from about 4 bp to about 150 bp, preferably from about 5 bp to about 50 bp, preferably from about 15 bp to about 50 bp, more preferably from about 6 bp to about 30 bp, more preferably from about 8 bp to about 20 bp in length.

In an embodiment of the first aspect or the second aspect, the scaffold cohesive end or the cross-linking cohesive end has a length of 4 nt or more, in this way it is advantageous for them to be in a stable cross-linked state under physiological conditions. Preferably, the scaffold cohesive end or the cross-linking cohesive end has a length of about 150 nt or less, preferably about 50 nt or less, more preferably about 30 nt or less, more preferably from about 30 nt to about 50 nt, more preferably about 20 nt or less; more preferably, the scaffold cohesive end or the cross-linking cohesive end has a length of from about 4 nt to about 30 nt, from about 4 nt to about 20 nt, more preferably from about 8 nt to about 12 nt.

In an embodiment of the first aspect or the second aspect, when the scaffold core is the same as the cross-linking core, the single-stranded nucleic acids bound to the scaffold core and the single-stranded nucleic acids bound to the cross-linking core are the same, e.g., the same L-deoxynucleic acids; when the number of single-stranded nucleic acids bound to the scaffold core and the number of single-stranded nucleic acids bound to the cross-linking core (both≥3) are the same, the scaffold unit is the same as the cross-linking unit. Thus, in an embodiment of the first aspect or the second aspect, the scaffold unit is the same as the cross-linking unit. In an embodiment of the first aspect or the second aspect, the scaffold unit is different from the cross-linking unit.

In an embodiment of the first aspect or the second aspect, the molar ratio of the scaffold units to the cross-linking units in the nucleic acid hydrogel is from about 2:1 to about 1:3, preferably from about 1:1 to about 1:2, more preferably about 1:1.5.

In an embodiment of the first aspect or the second aspect, the scaffold units and the cross-linking units are cross-linked by the way of base complementary pairing of the scaffold cohesive ends and the cross-linking cohesive ends, thereby forming a three-dimensional spatial network structure. Preferably, the scaffold units, the cross-linking units, and the three-dimensional spatial network structure are in a stable cross-linked state under physiological conditions (e.g., 37°C, pH 7.0-7.4, 0.9 wt% NaCl, isotonic).

In an embodiment of the first aspect or the second aspect, the solid content of the nucleic acids in the nucleic acid hydrogel formulation is from about 0.3 wt% to about 5 wt%, relative to the weight of the nucleic acid hydrogel formulation. Preferably, the solid content of the nucleic acids is from about 0.3 wt% to about 3.7 wt%; more preferably from about 1 wt% to about 3.5 wt%; more preferably from about 2 wt% to about 3.5 wt%; or from about 3 wt% to about 3.5 wt%. Although the nucleic acid hydrogel with a higher solid content has a higher storage modulus, intraocular injection will be more difficult. Moreover, the higher the solid content in the nucleic acid hydrogel, the longer the storage time of the nucleic acid hydrogel in eyes. However, longer storage time of the nucleic acid hydrogel in the eyes is not always better. The nucleic acid hydrogel generally needs to be degraded after stored in the eyes for about 3-6 months. Furthermore, a higher solid content may cause the nucleic acid hydrogel to become opaque after injection, generate air bubbles, and in turn affect refraction. Therefore, from about 3 wt% to about 3.5 wt% solid content of nucleic acids is suitable and more preferred for retinal reattachment applications, considering the difficulty of injection, degradation time, refraction, and the like.

In an embodiment of the first aspect or the second aspect, the single-stranded nucleic acids have a purity of about 90% or more, preferably a purity of about 95% or more, such as a purity of about 95%, about 96%, about 97%, about 98%, or about 99%. The single-stranded nucleic acids used in the present invention can be synthesized by a standard phosphoramidite DNA solid-phase synthesis method, separated and purified by a reversed-phase high performance liquid chromatography, and their purity can be characterized by LC-MS.

In an embodiment of the first aspect or the second aspect, the scaffold unit or cross-linking unit of the nucleic acid hydrogel may comprise a CpG sequence. The CpG sequence is a palindromic sequence having cytosine-guanine dinucleotide (CpG) as a core, with 2 purines at the 5' end and 2 pyrimidines at the 3' end, that is, 5'-PurPur-CG-PyrPyr-3'. The CpG sequence can be recognized by mammalian cells, thereby triggering a series of defense mechanisms in an organism, including complement activation, phagocytosis, and expression of proinflammatory cytokine genes. Currently known CpG sequence having strong immunostimulatory effects includes, for example, 5'-TCCATGACGTTCCT GACGTT-3' (SEQ ID NO: 1), and the like.

In an embodiment of the first aspect or the second aspect, the scaffold unit has sequences 5'-CGATTGACTCTCCACGCTGTCCTAACCATGACCGTCGAAG-3' (SEQ ID NO: 2), 5'-CGATTGACTCTCCTTCGACGGTCATGTACTAGATCAGAGG-3' (SEQ ID NO: 3), and 5'-CGATTGACTCTCCCTCTGATCTAGTAGTTAGGACAGCGTG-3' (SEQ ID NO: 4).

In an embodiment of the first aspect or the second aspect, the cross-linking unit has sequences 5'-GAGAGTCAATCGTCTATTCGCATGAGAATTCCATTC ACCGTAAG -3' (SEQ ID NO: 5), and 5'-GAGAGTCAATCGCTTACGG TGAATGGAATTCTCATGCGAATAGA-3' (SEQ ID NO: 6).

In an embodiment of the first aspect or the second aspect, the nucleic acid hydrogel has a suitable mechanical strength, for example, its mechanical strength may be about 0.1 Pa or more, preferably about 1 Pa or more, more preferably about 10 Pa or more, preferably about 10,000 Pa or less, more preferably about 1,000 Pa or less.

In an embodiment of the first aspect or the second aspect, the nucleic acid hydrogel has a predominant storage modulus G' relative to the loss modulus G" at all strain values from about 0.1 rad/s to about 100 rad/s, as measured by the frequency sweep at about 25°C and at a strain amplitude of about 1% strain. Preferably, at a strain value of about 0.1 rad/s to about 100 rad/s, the ratio of the storage modulus G' to the loss modulus G" is greater than about 2, more preferably greater than about 10. In some embodiments, the nucleic acid hydrogel has a storage modulus (G') that is more than or equal to about 500 Pa and not less than about 1,500 Pa.

In an embodiment of the first aspect or the second aspect, the hydrogel of the present invention may have desirable stability, for example, its structure may be stably maintained for about 24 hours, preferably about 36 hours, preferably about 48 hours, or longer, in the presence of a restriction endonuclease.

In an embodiment of the first aspect or the second aspect, the buffer is PBS buffer 1 comprising about 0.20 g KCl, about 8.00 g NaCl, about 0.20 g KH₂PO₄, and about 2.08 g Na₂HPO₄•12H₂O. In an embodiment of the first aspect or the second aspect, the buffer is PBS buffer 2 comprising about 0.20 g KCl, about 8.00 g NaCl, about 0.24 g KH₂PO₄, about 3.63 g Na₂HPO₄•12H₂O, and a pH adjusting agent used to adjust the pH value to about 7.4.

The PBS buffer 1 is prepared by adding about 0.20 g KCl, about 8.00 g NaCl, about 0.20 g KH₂PO₄, about 2.08 g Na₂HPO₄•12H₂O into 1 L water, mixing and dissolving, followed by sterile filtration through a 0.22 µm filter membrane. In one embodiment, the PBS buffer 1 has a pH value of about 7.4. In one embodiment, the PBS buffer 1 has an osmolality of about 277 mOsmol/kg.

The PBS buffer 2 is prepared by adding about 0.20 g KCl, about 8.00 g NaCl, about 0.24 g KH₂PO₄, about 3.63 g Na₂HPO₄•12H₂O into about 900 mL water, adjusting the pH to about 7.4 with about 1 mol/L HCl, and making the volume up to 1 L, followed by sterile filtration through a 0.22 µm filter membrane. In one embodiment, the PBS buffer 2 has a pH value of about 7.4. In one embodiment, the PBS buffer 2 has an osmolality of from about 285 mOsmol/kg to about 310 mOsmol/kg. In one embodiment, the PBS buffer 2 has an osmolality of about 288 mOsmol/kg. In the present invention, the pH value of the PBS buffer 2 remains stable after standing for one week.

The osmolality and pH stability of the PBS buffer 1 and the PBS buffer 2 are slightly different, and after standing for one week, the pH of the buffer 1 fluctuates, while the pH of PBS buffer 2 is stable.

In an embodiment of the first aspect or the second aspect, the pH adjusting agent is an HCl solution or a NaOH solution. Preferably, the pH adjusting agent is an HCl solution or a NaOH solution that makes the pH value from 7 to 7.4. The concentration of the HCl solution or the NaOH solution is generally from 0.01 mol/L to 1 mol/L, preferably 0.01 mol/L, to maintain a suitable pH and osmolality.

In an embodiment of the second aspect, the method further comprises subjecting the formulated scaffold unit assembly solution and the cross-linking unit assembly solution to sterile filtration prior to assembly into a gel. In one embodiment, the sterile filtration comprises passing the formulated scaffold unit assembly solution and cross-linking unit assembly solution through one or more filter membranes, respectively. In one embodiment, the sterile filtration comprises passing the formulated scaffold unit assembly solution and cross-linking unit assembly solution through one 0.45 µm and two 0.22 µm filter membranes, respectively. In one embodiment, the filter membrane may be any suitable polymer filter membrane or ceramic filter membrane.

In an embodiment of the second aspect, the assembly into a gel is carried out at 40°C-65°C, preferably at 45°C-55°C (i.e. the temperature for the assembly into a gel). The inventors of the present invention have found that gelation at temperatures above room temperature can quickly yield clear and transparent hydrogels, whereas at room temperature or lower temperatures, the resulting hydrogels are cloudy and bubbly.

In an embodiment of the second aspect, the assembly into a gel comprises heating and stirring the formulated scaffold unit assembly solution at the gelation temperature described above, followed by adding the formulated cross-linking unit assembly solution and keeping warm for a period of time, such as from about 0.5 hour to about 5 hours or from about 1 hour to about 2 hours. In an embodiment of the second aspect, the assembly into a gel comprises simultaneously heating and stirring the formulated scaffold unit assembly solution and the formulated cross-linking unit assembly solution at the gelation temperature described above, for example, for from about 0.5 hour to about 5 hours or from about 1 hour to about 2 hours.

In an embodiment of the second aspect, the assembly into a gel is carried out under a vacuum condition.

In an embodiment of the second aspect, the method further comprises filling the formed hydrogel into a syringe at a suitable temperature after the assembly into a gel. In an embodiment of the second aspect, the filling is carried out at a temperature below the gelation temperature but above room temperature after the formed hydrogel is stabilized; and if the filling is carried out at a temperature below room temperature, air bubbles will form in the filled hydrogel. In an embodiment of the second aspect, the filling is carried out at about 35°C to about 40°C.

In an embodiment of the first and second aspects, the nucleic acid hydrogel formulation has one or more of the following properties: 1) from about 96% to about 99% water content; 2) a pH value of from about 7.0 to about 7.4; 3) a density of from about 1.033 g/cm³ to about 1.0090 g/cm³; and 4) a refractive index of from about 1.3300 to about 1.3395. In one embodiment, the nucleic acid hydrogel formulation has all of the properties described above.

A third aspect relates to a method for treating an ophthalmic disease in a subject in need thereof, the method comprising injecting a therapeutically effective amount of a nucleic acid hydrogel formulation of the present invention into an eye of the subject; alternatively, relates to the use of the nucleic acid hydrogel of the present invention for the preparation of a medicament or reagent for the treatment of an ophthalmic disease.

In one embodiment, the nucleic acid hydrogel formulation is injected into the eyes after vitrectomy.

In one embodiment, the ocular disease is selected from retinal detachment, macular holes, a diabetic retinopathy-related complication, and ocular trauma of the posterior segment.

The nucleic acid hydrogel formulation of the present invention is capable of forming a hydrogel with a suitable strength in the eyes, which has one or more of the following advantages:
a) a shear thinning behavior so that it can be safely injected into the eyes;
b) an appropriate mechanical strength and rapid recovery property, so that it can be used for the reattachment of a retina with indentation and the maintenance of eyeball shape;
c) physical properties similar to the human vitreous body, such as the pH value, density, and refractive index described above, etc.;
d) an excellent biocompatibility;
e) a desirable stability, for example, stable structural retention for 48 hours, 4-8 weeks or longer in the presence of a restriction endonuclease, such as for 3-6 months in ocular tissue filling;
f) in vivo stability and safety, for example, no toxic or inflammatory response in the eyes; no complications such as turbid vitreous chamber, bleeding, retinal detachment, etc.

Therefore, the nucleic acid hydrogel formulation of the present invention is used for injection and filling to support the retina with indentation after a PPV surgery in patients with various retinal detachment, thereby achieving the use for assisting retinal reattachment.

### BRIEF DESCRIPTION OF DRAWINGS

FIG. 1 shows the purities of the single-stranded nucleic acids in Example 1 as determined by LC-MS.
FIG. 2 shows the measurement results of the storage modulus (G') and the loss modulus (G'') of the nucleic acid hydrogel formulation 1.
FIG. 3 shows the transparency measurement results of the nucleic acid hydrogel formulation 1.
FIG. 4 shows the purities of the single-stranded nucleic acids in Example 2 as determined by LC-MS.
FIG. 5 shows the measurement results of the storage modulus (G') and the loss modulus (G'') of the nucleic acid hydrogel formulation 2.
FIG. 6 shows the measurement results of the storage modulus (G') and the loss modulus (G'') of the nucleic acid hydrogel formulation 3.
FIG. 7 shows the transparency measurement results of the nucleic acid hydrogel formulation 3.
FIG. 8 shows the appearance comparison of the gels formed by gelation at different temperatures.
FIG. 9 shows the study on the properties of hydrogel formulations under different solid contents.
FIG. 10 shows B-mode ultrasound images confirming the successful construction of retinal detachment model in rabbit eyes.
FIG. 11 shows B-mode ultrasound images comparing the effects of nucleic acid hydrogel formulations and traditional silicone oil in the retinal detachment model.

### DETAILED DESCRIPTION

The scientific and technological terms mentioned in this specification have the same meanings as those commonly understood by those skilled in the art, and in case of any conflict, the definitions in this specification shall prevail.

As used in the present application, "comprising/comprises/comprised" is to be construed as indicating the presence of stated features, integers, steps, or components when referred to, but does not exclude the presence or addition of one or more features, integers, steps, or components, or groups thereof. Furthermore, the terms "by", "comprising/ comprises/comprised of", "including/includes/included", "involving/involves/involved", and "such as" are each used in their open non-limiting sense and are used interchangeably. In addition, the term "comprising/comprises/comprised" is intended to include the examples and aspects encompassed by the terms "consisting essentially of" and "consisting of". Similarly, the term "consisting essentially of" is intended to include the examples encompassed by the term "consisting of".

As used in this specification and the appended claims, the singular forms "a (or an)" and "the" include plural referents unless the context clearly indicates otherwise.

As used in the present application, the term "effective amount" refers to an amount sufficient to achieve a desired modification of a physical property of a composition or material. By way of example, an "effective amount" of a monomer refers to an amount sufficient to achieve a desired improvement in a property (e.g., a desired release rate of an antioxidant or viscoelasticity) modulated by the components in a formulation. The particular level in terms of wt% in the composition required as an effective amount will depend on a variety of factors, including the amount and type of a monomer, the amount and type of a polymer (e.g., acrylamide), the amount of an antioxidant, and the desired release kinetics.

As used in the present application, the term "room temperature" refers to a temperature of about 25°C.

As used in the present application, the term "about" refers to a set composed of all values within the range of ±5% of the numerical value given thereafter.

As used in the present application, the term "optional" or "optionally" means that the subsequently described event or situation may or may not occur, and that the description includes circumstances in which the event or situation occurs and circumstances in which it does not occur.

### EXAMPLES

### Materials:

1. Single-stranded DNA: DNA sequences were synthesized by the standard phosphoramidite DNA solid-phase synthesis method, separated and purified by the reversed-phase high performance liquid chromatography, and their purities can be characterized by LC-MS. Specific DNA sequences are shown in Table 1 below.

**Table 1. Information on the DNA sequences used to construct DNA hydrogels**

| **Name** | **nt** | **ε** | **M** | **Sequence** |
|---|---|---|---|---|
| **Y1** | 40 | 370600 | 12177 | 5'-CGATTGACTCTCCACGCTGTCCTAACCATGACCGTCGAAG-3' |
| **SEQ ID NO: 2** | | | | |
| **Y2** | 40 | 383000 | 12287 | 5'-CGATTGACTCTCCTTCGACGGTCATGTACTAGATCAGAGG-3' |
| **SEQ ID NO: 3** | | | | |
| **Y3** | 40 | 378300 | 12278 | 5'-CGATTGACTCTCCCTCTGATCTAGTAGTTAGGACAGCGTG-3' |
| **SEQ ID NO: 4** | | | | |
| **L1C** | 44 | 433300 | 13515 | 5'-GAGAGTCAATCGTCTATTCGCATG**AGAATTC**CATTCACCGTAAG-3' |
| **SEQ ID NO: 5** | | | | |
| **L2C** | 44 | 444200 | 13644 | 5'-GAGAGTCAATCGCTTACGGTGAATG**GAATTC**TCATGCGAATAGA-3' |
| **SEQ ID NO: 6** | | | | |

ε represents the absorbance coefficient, in L/mol· cm. Sequences Y1, Y2, and Y3 form a Y-scaffold unit. Sequences L1C and L2C form a cross-linking unit. The cohesive ends of the DNA sequences are underlined, and the recognition sequence of EcoR I restriction endonuclease is indicated in bold.

### 2. Buffer

The present invention uses the PBS buffer 1 and PBS buffer 2 described below.

The PBS buffer 1 was prepared by adding 0.20 g KCl, 8.00 g NaCl, 0.20 g KH₂PO₄, 2.08 g Na₂HPO₄•12H₂O into 1 L water, mixing and dissolving, followed by sterile filtration through a 0.22 µm filter membrane to give a PBS solution with a pH value of 7.4 and an osmolality of about 277 mOsmol/kg as measured.

The PBS buffer 2 was prepared by adding about 0.20 g KCl, about 8.00 g NaCl, about 0.24 g KH₂PO₄, about 3.63 g Na₂HPO₄•12H₂O into about 900 mL water, adjusting the pH to about 7.4 with about 1 mol/L HCl, and making the volume up to 1 L, followed by sterile filtration through a 0.22 µm filter membrane. The buffer had an osmolality of about 288 mOsmol/kg as measured.

### Example 1: Nucleic Acid Hydrogel Formulation 1

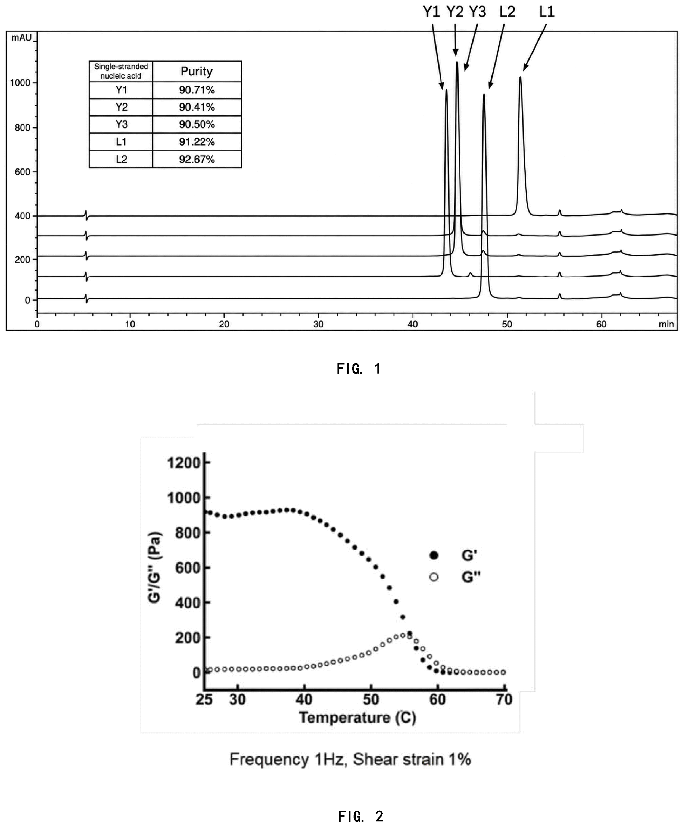

The method for preparing the nucleic acid hydrogel formulation of the present invention comprises the following standard steps,
1) The scaffold unit Y and the cross-linking unit L as shown in Table 1 were used, wherein the purities of the single-stranded nucleic acids were 90% as determined by LC-MS, as shown in FIG. 1.
2) The assembly solutions were formulated.

**Table 2: Prescription information of the standard steps of the nucleic acid hydrogel**

| Composition of prescription | Prescribed amount (1 L) |
|---|---|
| Y1 | 428 µmol |
| Y2 | 428 µmol |
| Y3 | 428 µmol |
| PBS solution | 0.5 L |
| L1C | 642 µmol |
| L2C | 642 µmol |
| PBS solution | 0.5 L |

The prescribed amounts of Y1, Y2, and Y3 were respectively weighed into formulation tank A, and then the prescribed amount of PBS solution 1 was added, and they were stirred at room temperature until complete dissolution. The pH of the system was detected by an online pH monitor, and the pH was adjusted to 7.2 with a NaOH solution, and the Y assembly solution was prepared at room temperature.

The prescribed amounts of L1C and L2C were respectively weighed into formulation tank B, and then the prescribed amount of PBS solution 1 was added, and they were stirred at room temperature until complete dissolution. The pH of the system was detected by an online pH monitor, and the pH was adjusted to 7.2 with a NaOH solution, and the L assembly solution was prepared at room temperature.

### 3) Sterile filtration

The Y assembly solution and the L assembly solution were subjected to sterile filtration through one 0.45 µm and two 0.22 µm PES (polyethersulfone) filter membranes, respectively.

### 4) Assembly into a gel

The filtered Y assembly solution was transferred into a mixing tank, heated to 55°C, and L assembly solution was added with stirring, and the mixture was kept warm for 2 hours, during which time vacuum degassing was performed.

### 5) Filling

After the formed gels were stable, they were cooled to 40°C, filled into pre-filled syringes, and then packaged.

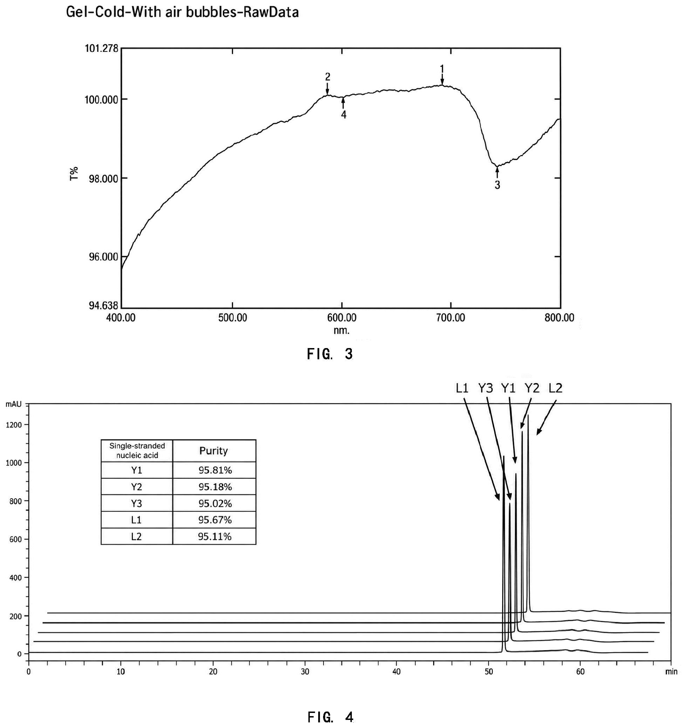

The properties of the nucleic acid hydrogel formulation 1 are as follows:
1. The detected solid content of nucleic acids was 3.0 wt% relative to the nucleic acid hydrogel formulation;
2. Visually, the nucleic acid hydrogel formulation 1 was a colorless transparent gel;
3. The storage modulus (G') and loss modulus (G'') of the nucleic acid hydrogel formulation 1 were measured by a rotary rheometer, and the results are shown in FIG. 2: the storage modulus (G') and loss modulus (G'') were measured in the frequency range of 0.01 Hz-10 Hz; and G' was greater than G", indicating that the hydrogel was successfully formed, while G' exceeded 500 Pa, indicating that the hydrogel was a strong gel;
4. The transparency of the nucleic acid hydrogel formulation 1 was measured by ultraviolet spectrophotometry: that is, the light transmittances T% by scanning at 400 nm-800 nm were all greater than 90%, as shown in FIG. 3, indicating that the hydrogel was transparent in the visible light region.
5. The pH value of the nucleic acid hydrogel formulation 1:
   a) was pH 7.36 after preparation;
   b) gradually dropped to pH 6.84 after two weeks.

### Example 2: Nucleic Acid Hydrogel Formulation 2

The nucleic acid hydrogel 2 was prepared in a similar manner to Example 1, except that the purities of the single-stranded nucleic acids used were 95% as determined by LC-MS, as shown in FIG. 4; in addition, the pH was adjusted to 7.2 using more NaOH solution when preparing the Y assembly solution at room temperature.

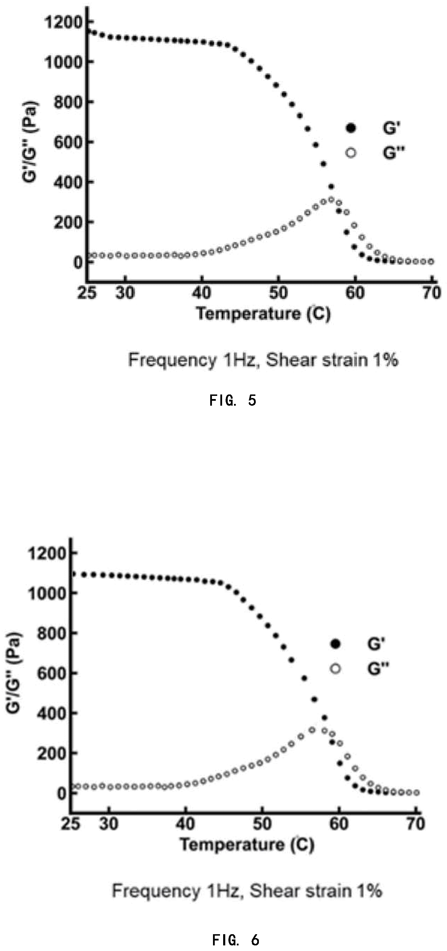

The properties of the nucleic acid hydrogel formulation 2 are as follows:
1. The detected solid content of nucleic acids was 3.0 wt% relative to the nucleic acid hydrogel formulation;
2. Visually, the nucleic acid hydrogel formulation 2 was a colorless transparent gel;
3. The storage modulus (G') and loss modulus (G'') of the nucleic acid hydrogel formulation 2 were measured by a rotary rheometer, and the results are shown in FIG. 5: the storage modulus (G') and loss modulus (G'') were measured in the frequency range of 0.01 Hz-10 Hz; and G' was greater than G", indicating that the hydrogel was successfully formed, while G' exceeded 500 Pa, indicating that the hydrogel was a strong gel;

The experimental results suggested that no obvious difference was observed by naked eyes in the nucleic acid hydrogel formulations made of the single-stranded nucleic acids with different purities; however, after the single-stranded nucleic acids with purities of 95% or more were formulated into an assembly solution, the pH was more acidic and needed to be adjusted with more NaOH aqueous solution; and the prepared hydrogel also had a larger storage modulus, increasing to 1,200 Pa, which could be predicted to have better filling and supporting performance.

### Example 3: Nucleic Acid Hydrogel Formulation 3

The nucleic acid hydrogel 3 was prepared in a similar manner to Example 1, except that the PBS buffer 2 was used instead of the PBS buffer 1.

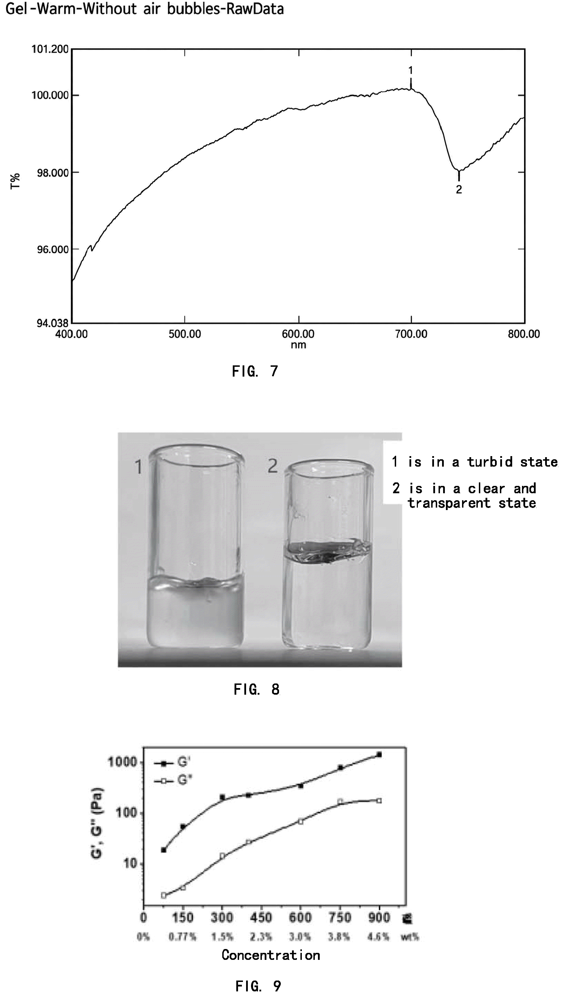

The properties of the nucleic acid hydrogel formulation 3 are as follows:
1. The pH value of the nucleic acid hydrogel formulation 3:
   a) was pH 7.25 after preparation;
   b) gradually dropped to pH 7.36 after two weeks.
2. The storage modulus (G') and loss modulus (G'') of the nucleic acid hydrogel formulation 3 were measured by a rotary rheometer, and the results are shown in FIG. 6: the storage modulus (G') and loss modulus (G'') were measured in the frequency range of 0.01 Hz-10 Hz; and G' was greater than G", indicating that the hydrogel was successfully formed, while G' exceeded 500 Pa, indicating that the hydrogel was a strong gel;
3. The transparency of the nucleic acid hydrogel formulation 3 was measured by ultraviolet spectrophotometry: that is, the light transmittances T% by scanning at 400 nm-800 nm were all greater than 90%, as shown in FIG. 7, indicating that the hydrogel was transparent in the visible light region.

By measuring the pH of the hydrogel at different times, it was found that the nucleic acid hydrogel formulation 3 prepared by utilizing the buffer 2 can maintain the pH value between 7.0 and 7.4 for a long time, which meets the standard of ophthalmic formulation; whereas, the pH value of the nucleic acid hydrogel formulation 1 prepared by utilizing the buffer 1 gradually decreased from 7.36 after preparation to 6.84 after two weeks.

### Example 4: Effects of Gelation at Different Temperatures on the Properties of Nucleic Acid Hydrogels

The following nucleic acid hydrogel formulations were prepared in a similar manner to Example 3, except for the gelation temperature:
Example 4a: Gelation at 5°C
Example 4b: Gelation at 25°C
Example 4c: Gelation at 37°C
Example 4d: That is, in Example 3, gelation at 55°C
Example 4e: Gelation at 65°C

The final physical properties and rheological properties of the nucleic acid hydrogel formulations prepared at different temperatures did not vary significantly. However, the experimental results suggested that the lower the temperature, the longer the time of gelation; in the 5°C and 25°C experimental groups, the gels appeared in a turbid state with a large number of air bubbles after the assembly solutions were mixed, and gradually became clear and transparent after 24 hours, but the air bubbles could not disappear by themselves; for the 37°C experimental group, the gel appeared in a turbid state with a large number of air bubbles after the assembly solutions were mixed, and after standing for 1-2 hours, gradually became clear and transparent, and the air bubbles disappeared; at 55°C and 65°C, after the assembly solutions were mixed, the system was in an almost liquid state without air bubble formation, and clear and transparent gels were formed as the temperature decreased to below 55°C. FIG. 8 shows the turbid (Example 4a, left panel) and clear (Example 4d, right panel) states of the hydrogels prepared at different gelation temperatures, wherein the hydrogel formed at room temperature was in a turbid state, while the hydrogel formed at temperatures higher than room temperature, for example, 55°C or 65°C, was clear and transparent.

It can be seen from the above data that appropriately increasing the temperature can accelerate the stability of the gelation system and eliminate the air bubbles, but considering the possibility of nucleic acid degradation under high temperatures for a long time, the gelation can finally be carried out at 40°C-65°C, preferably it is most suitable for the gelation to be carried out at 45°C-55°C.

### Example 5: Effects of the Solid Content of Nucleic Acids on the Properties of Nucleic Acid Hydrogel Formulations

The following nucleic acid hydrogel formulations were prepared in a similar manner to Example 3, except for the different solid contents of nucleic acids:
Example 5a: The solid content of nucleic acids was 0.385 wt%;
Example 5b: The solid content of nucleic acids was 0.77 wt%;
Example 5c: The solid content of nucleic acids was 1.5 wt%;
Example 5d: The solid content of nucleic acids was 2.0 wt%;
Example 5e: That is, in Example 3, the solid content of nucleic acids was 3.0 wt%;
Example 5f: The solid content of nucleic acids was 3.8 wt%;
Example 5g: The solid content of nucleic acids was 4.6 wt%.

The effects of different solid contents on the properties of the prepared nucleic acid hydrogel formulations were then tested. The results are as shown in FIG. 9. The storage modulus of the prepared nucleic acid hydrogels increased sequentially from 20 Pa to 1,371 Pa as the solid content of the nucleic acids increased from 0.385 wt% to 4.6 wt%. At the initial stage of the injection and filling of ocular vitreous chamber, the supporting performance would reach the best. With the secretion and dilution of aqueous humor, the hydrogel at the site in contact with the aqueous humor may be diluted and dissolved by the aqueous humor and taken out by the aqueous humor circulation. The solid content of DNA would gradually decrease, and the storage modulus of the hydrogel would also decrease, until all of the hydrogel became liquid and could not form a gel.

Although the nucleic acid hydrogel with a higher solid content has a higher storage modulus, intraocular injection will be more difficult. Moreover, the higher the solid content in the nucleic acid hydrogel, the longer the storage time of the nucleic acid hydrogel in the eyes. However, longer storage time of the nucleic acid hydrogel in the eyes is not always better. The nucleic acid hydrogel generally needs to be degraded after stored in the eyes for about 3-6 months. Furthermore, a higher solid content may cause the nucleic acid hydrogel to become opaque after injection, generate air bubbles, and in turn affect refraction. Therefore, from 3 wt% to 3.5 wt% solid content of nucleic acids is suitable and more preferred for retinal reattachment applications, considering the difficulty of injection, degradation time, refraction, and the like.

### Example 6: Auxiliary Function of the Filling of the Nucleic Acid Hydrogel Formulations on the Retinal Reattachment

This example studied and evaluated the effects of injection and filling the nucleic acid hydrogel formulation 3 prepared in Example 3 after the PPV surgery in the retinal detachment model on the retinal reattachment.

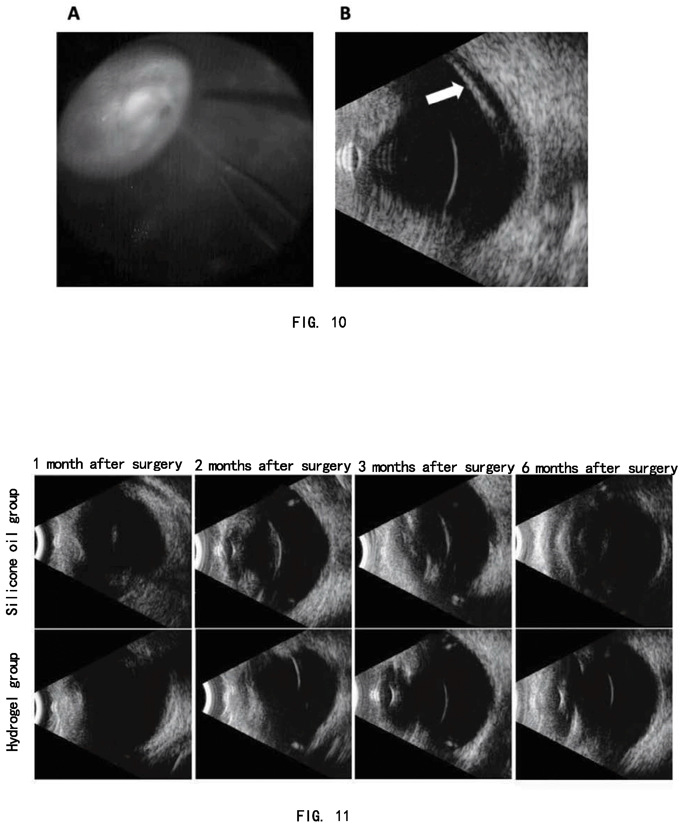

An animal model of retinal detachment was constructed using chinchilla rabbits. FIG. 10 shows the construction of a rabbit eye retinal detachment model. FIG. 10A shows the retina detached under the impact of water flow. After the retina was temporarily reattached with the hole closed by laser, the hydrogel or the traditional silicone oil was not used for filling. The B-mode ultrasound of the eyes was reviewed 1 month after modeling, and the results revealed that the retina was still in a detached state (FIG. 10B).

FIG. 10A. Retinal detachment formation (the white area is the detached retina, the central red dot is a hole); FIG. 10B. The B-mode ultrasound revealed that the retina was still detached at 1 month after surgery (the white arrow indicates the detached retina).

Twelve chinchilla rabbits were randomly divided into the hydrogel group and the silicone oil group, with 6 rabbits in each group. The right eye was used as the surgical eye for all the rabbits. The retinal detachment model was first constructed, and then the retina was reattached, and then about 1 ml of hydrogel or silicone oil was injected into the vitreous chamber, respectively. As shown in FIG. 11, B-mode ultrasound examination was performed at 1 month, 2 months, 3 months, and 6 months after surgery. It was found that the retina of the eyes of all rabbits in the hydrogel group and the silicone oil group was in place at each observation time point.

The retinal reattachment status was observed by injecting the hydrogel formulations into the rabbit retinal detachment model. The results showed that the retinas of all rabbit eyes in the hydrogel group and the traditional silicone oil group were in place within 6 months. Compared with silicone oil, the use of nucleic acid hydrogel formulation does not require the patient to maintain a prone position for 2-3 weeks, which greatly alleviates the distress of the patient; and due to the characteristic that nucleic acids will be degraded by nucleases in vivo, secondary surgical removal is not required.

From these two experimental results, it can be seen that the nucleic acid hydrogel formulations achieved the same effect of supporting with indentation as the silicone oil, providing an alternative option for clinical treatment of the retinal detachment.

Although the examples of the present invention have been shown and described above, it can be appreciated that the above-mentioned examples are exemplary and should not be construed as limiting the present invention. Those of ordinary skill in the art may make changes, modifications, substitutions, and variations to the above-mentioned examples within the scope of the present invention.

## Claims

1. A nucleic acid hydrogel formulation suitable for use in retinal reattachment, the nucleic acid hydrogel formulation comprising
a scaffold unit comprising a scaffold core and at least three single-stranded nucleic acids bound to the scaffold core, each single-stranded nucleic acid having at least one scaffold cohesive end;
a cross-linking unit comprising a cross-linking core and at least two single-stranded nucleic acids bound to the cross-linking core, each single-stranded nucleic acid having at least one cross-linking cohesive end; and
the scaffold units and the cross-linking units being cross-linked by the way of base complementary pairing of the scaffold cohesive ends and the cross-linking cohesive ends to form a nucleic acid hydrogel having a three-dimensional spatial network structure;
a buffer; and
optionally a pH adjusting agent.

2. A method for preparing a nucleic acid hydrogel formulation, the method comprising:
a) formulating a scaffold unit assembly solution and a cross-linking unit assembly solution; and
b) assembling the formulated scaffold unit assembly solution and the cross-linking unit assembly solution into a gel at a temperature higher than room temperature.

3. The nucleic acid hydrogel formulation of claim 1 or the method of claim 2, wherein the scaffold unit comprises a scaffold core and at least three single-stranded nucleic acids bound to the scaffold core, each single-stranded nucleic acid having at least one scaffold cohesive end; preferably, the scaffold core is a nucleic acid, specifically may be a D-nucleic acid or an L-nucleic acid, more specifically may be a D-DNA or an L-DNA; preferably, the nucleic acid serving as the scaffold core has a complementary pairing region which may be from 4 bp to 150 bp, preferably from 5 bp to 50 bp, more preferably from 6 bp to 30 bp, more preferably from 8 bp to 20 bp in length.

4. The nucleic acid hydrogel formulation of claim 1 or the method of claim 2, wherein the cross-linking unit comprises a cross-linking core and at least two single-stranded L-deoxynucleic acids bound to the cross-linking core, each single-stranded L-deoxynucleic acid having at least one cross-linking cohesive end; preferably, the cross-linking core may be a deoxynucleic acid, specifically may be a D-nucleic acid or an L-nucleic acid, more specifically may be a D-DNA or an L-DNA.

5. The nucleic acid hydrogel formulation of claim 4 or the method of claim 4, wherein the deoxynucleic acid serving as the cross-linking core has a complementary pairing region which may be from 4 bp to 150 bp, preferably from 5 bp to 50 bp, preferably from 15 bp to 50 bp, more preferably from 6 bp to 30 bp, more preferably from 8 bp to 20 bp in length.

6. The nucleic acid hydrogel formulation of claim 1 or the method of claim 2, wherein the scaffold cohesive end or the cross-linking cohesive end has a length of from 4 nt to 30 nt, from 4 nt to 20 nt, more preferably from 8 nt to 12 nt.

7. The nucleic acid hydrogel formulation of claim 1 or the method of claim 2, wherein the scaffold unit is the same as the cross-linking unit.

8. The nucleic acid hydrogel formulation of claim 1 or the method of claim 2, wherein the scaffold unit is different from the cross-linking unit.

9. The nucleic acid hydrogel formulation of claim 1 or the method of claim 2, wherein the molar ratio of the scaffold units to the cross-linking units in the nucleic acid hydrogel is from 2:1 to 1:3, preferably from 1:1 to 1:2, more preferably 1:1.5.

10. The nucleic acid hydrogel formulation of claim 1 or the method of claim 2, wherein the scaffold units and the cross-linking units are cross-linked by the way of base complementary pairing of the scaffold cohesive ends and the cross-linking cohesive ends, thereby forming a three-dimensional spatial network structure.

11. The nucleic acid hydrogel formulation of claim 1 or the method of claim 2, wherein the solid content of the nucleic acids in the nucleic acid hydrogel formulation is from 0.3 wt% to 5 wt% relative to the weight of the nucleic acid hydrogel formulation; preferably, the solid content of the nucleic acids is from 0.3 wt% to 3.7 wt%; more preferably from 1 wt% to 3.5 wt%; more preferably from 2 wt% to 3.5 wt%; or from 3 wt% to 3.5 wt%.

12. The nucleic acid hydrogel formulation of claim 1 or the method of claim 2, wherein the single-stranded nucleic acid has a purity of 90% or more, preferably a purity of 95% or more, for example a purity of 95%, 96%, 97%, 98%, or 99%.

13. The nucleic acid hydrogel formulation of claim 1 or the method of claim 2, wherein the scaffold unit or the cross-linking unit of the nucleic acid hydrogel may comprise a CpG sequence.

14. The nucleic acid hydrogel formulation of claim 1 or the method of claim 2, wherein the scaffold unit has sequences 5'-CGATTGACTCTCCA CGCTGTCCTAACCATGACCGTCGAAG-3' (SEQ ID NO: 2), 5'-CGATTGACTCTC CTTCGACGGTCATGTACTAGATCAGAGG-3' (SEQ ID NO: 3), and 5'-CGATTGACT CTCCCTCTGATCTAGTAGTTAGGACAGCGTG-3' (SEQ ID NO: 4); and
the cross-linking unit has sequences 5'-GAGAGTCAATCGTCTATTCGCATGAG AATTCCATTCACCGTAAG-3' (SEQ ID NO: 5) and 5'-GAGAGTCAATCGCTTA CGGTGAATGGAAT TCTCATGCGAATAGA-3' (SEQ ID NO: 6).

15. The nucleic acid hydrogel formulation of claim 1 or the method of claim 2, wherein the nucleic acid hydrogel produced has a mechanical strength of 0.1 Pa or more, preferably 1 Pa or more, more preferably 10 Pa or more, preferably 10,000 Pa or less, more preferably 1,000 Pa or less.

16. The nucleic acid hydrogel formulation of claim 1 or the method of claim 2, wherein the nucleic acid hydrogel produced has a predominant storage modulus G' relative to the loss modulus G" at all strain values from 0.1 rad/s to 100 rad/s, as measured by frequency sweep at 25°C and at a strain amplitude of 1% strain; preferably, at a strain value of 0.1 rad/s to 100 rad/s, the ratio of storage modulus G' to loss modulus G" is greater than 2; more preferably greater than 10.

17. The nucleic acid hydrogel formulation of claim 1 or the method of claim 2, wherein the buffer is PBS buffer 1 comprising about 0.20 g KCl, about 8.00 g NaCl, about 0.20 g KH₂PO₄, and about 2.08 g Na₂HPO₄•12H₂O; alternatively, the buffer is PBS buffer 2 comprising about 0.20 g KCl, about 8.00 g NaCl, about 0.24 g KH₂PO₄, about 3.63 g Na₂HPO₄•12H₂O, and a pH adjusting agent which adjusts the pH to about 7.4.

18. The nucleic acid hydrogel formulation of claim 1 or the method of claim 2, wherein the pH adjusting agent is an HCl solution or a NaOH solution; preferably, the pH adjusting agent is an HCl solution or a NaOH solution that makes the pH value from 7 to 7.4, and the concentration of the HCl solution or the NaOH solution is generally from 0.01 mol/L to 1 mol/L, preferably 0.01 mol/L.

19. The method of claim 2, wherein the method further comprises subjecting the formulated scaffold unit assembly solution and the cross-linking unit assembly solution to sterile filtration prior to assembly into a gel.

20. The method of claim 2, wherein the assembly into a gel is carried out at 40°C-65°C, preferably at 45°C-55°C.

21. The method of claim 2, wherein the assembly into a gel comprises heating and stirring the formulated scaffold unit assembly solution at the gelation temperature described above, followed by adding the formulated cross-linking unit assembly solution and keeping warm for a period of time, for example, for 0.5-5 hours or 1-2 hours.

22. The method of claim 2, wherein the assembly into a gel comprises simultaneously heating and stirring the formulated scaffold unit assembly solution and the formulated cross-linking unit assembly solution at the gelation temperature described above, for example, for 0.5-5 hours or 1-2 hours.

23. The method of claim 2, wherein the assembly into a gel is carried out under a vacuum condition.

24. The method of claim 2, wherein the method further comprises filling the formed hydrogel into a syringe at a suitable temperature after the assembly into a gel.

25. A method for treating an ophthalmic disease in a subject in need thereof, the method comprising injecting a therapeutically effective amount of the nucleic acid hydrogel formulation of the present invention into an eye of the subject; or use of the nucleic acid hydrogel of the present invention for the preparation of a medicament or reagent for the treatment of an ophthalmic disease.

26. The method of claim 25, wherein the nucleic acid hydrogel formulation is injected into an eye after vitrectomy.

27. The method of claim 25, the ocular disease is selected from retinal detachment, macular holes, a diabetic retinopathy-related complication, and ocular trauma of the posterior segment.
